# EUROPÄISCHE PATENTSCHRIFT

(11) **EP 2 283 082 B1**
(45) Veröffentlichungstag und Bekanntmachung des Hinweises auf die Patenterteilung: **28.09.2011**
(21) Anmeldenummer: 09757108.7
(22) Anmeldetag: 19.05.2009
(51) Int. Cl.: C09D 1/00, A61L 27/30

(54) **HYDROPHOBE BESCHICHTUNG UND VERFAHREN ZU DEREN HERSTELLUNG**
HYDROPHOBIC COATING AND PROCESS FOR PRODUCTION THEREOF
REVÊTEMENT HYDROPHOBE ET SON PROCÉDÉ DE PRODUCTION

(30) Priorität: 05.06.2008 DE 102008026988
(43) Veröffentlichungstag der Anmeldung: 16.02.2011
(73) Patentinhaber: Fachhochschule Kiel, 24149 Kiel (DE)
(72) Erfinder: ES-SOUNI, Mohammed, 24247 Mielkendorf (DE)
(74) Vertreter: Lobemeier, Martin Landolf
(86) Internationale Anmeldenummer: PCT/DE2009/000707
(87) Internationale Veröffentlichungsnummer: WO 2009/146674

(56) Entgegenhaltungen:
- WO-A2-2005/105304
- US-A1- 2004 202 890
- PAGE K.: "Titania and silver-titania composite films on glass" JOURNAL OF MATERIALS CHEMISTRY, Bd. 2007, Nr. 17, 3. November 2006 (2006-11-03), Seiten 95-104, XP002547161 DOI: 10.1039/b611740f

## Beschreibung

Die Erfindung betrifft eine Silber-Ionen enthaltende Titandioxid-Beschichtung, die über eine veränderbare Hydrophobizität verfügt, sowie ein Verfahren zu ihrer Herstellung.

Die Neigung einer Substanz sich mit Wasser zu mischen wird durch die Hydrophilie beziehungsweise den Gegensatz hierzu, die Hydrophobie beschrieben. Das Ausmaß der Hydrophobie einer Oberfläche wird durch die Hydrophobizität beschrieben. Ein physikalisches Maß für die Hydrophobizität einer Oberfläche ist der Kontaktwinkel, dessen Messung durch die DIN EN 828 beschrieben wird. Bei einem Kontaktwinkel von 0° bildet das Wasser auf der Festkörperoberfläche einen monomolekularen Film, während bei einem Kontaktwinkel von 180° ein Wassertropfen die Festkörperoberfläche nur an einem Punkt berührt. Bei einem geringen Kontaktwinkel nahe 0° besteht eine starke Wechselwirkung zwischen der Oberfläche und dem Wassertropfen, die Oberfläche wird als hydrophil bezeichnet. Bei Kontaktwinkeln um die 90° und darüber handelt es sich um hydrophobe Oberflächen, die Benetzbarkeit ist hier sehr gering. Bei noch größeren Kontaktwinkeln werden die Oberflächen als superhydrophob bezeichnet, bei Kontaktwinkeln von ca. 160° wird vom so genannten Lotuseffekt gesprochen.

Das Fliessverhalten eines strömenden Mediums wird durch die Hydrophobizität der umgebenden Gefäßwände mit bestimmt.

Für eine Vielzahl von Anwendungen z.B. medizinische Implantate, wie z.B. Stents, mikroskalige Reaktoren, Innenbeschichtungen von Aufbewahrungs und Reaktionsgefäßen ist die exakte, dauerhafte Einstellung der Hydrophobie/Hydrophilie auch auf kleinstem Raum von großer Bedeutung.

Insbesondere im Bereich der Medizintechnik ist eine einstellbare Hydrophobizität von größter Bedeutung, da hier auf kleinem Raum zum Einen das Vorbeiströmen von Körperflüssigkeiten ungehindert, optimal von Statten gehen soll, anderseits direkter Kontakt mit dem Körpergewebe vorhanden ist und hier ein Anwachsen des Implantates gewünscht ist. Es ist hier also gefordert, dass ein einzelnes Implantat durchaus verschiedene Hydrophobizität aufweist. Ein besonderer Vorteil wäre es, wenn während des Einsatzes des Implantates die Hydrophobizität exakt an den Patienten angepasst werden kann. Weiterhin kann die nachträgliche Strukturierung von dafür geeigneten Materialien/Schichten in benachbarte hydrophobe-hydrophile Bereiche für Anwendungen in der Milcro-Fluidik genutzt werden.

Die Auswahl geeigneter Träger für eine Beschichtung ist begrenzt, wenn die aufgetragenen Schichten durch thermische Behandlung oberhalb von 200 °C ausgehärtet werden müssen. Nicht jeder Träger ist für diese Temperaturen geeignet.

Die Kosten eines Herstellungsprozesses hängen unter anderem von der benötigten Energie und damit von der zur Durchführung des Prozesses notwendigen Heizleistung ab, eine Senkung der Herstellungstemperatur auf unter 200 °C spart Energiekosten.

In der Veröffentlichung "Titania and silver-titania composite films on glass-potent antimicrobial coatings" Page et. al.; J. Mater. Chem., 17, 95-104 werden ebenfalls mit geringen Mengen an Silberionen versetzte hydrophile Titandioxid-Schichten beschrieben. Die Schichten zeigen vor ihrer Belichtung Kontaktwinkel von 15° und keine antimikrobielle Wirkung, während der Belichtung mit UV-Licht kann eine nicht persistente (wir können vielleicht "persistent" durch "dauerhaft" ersetzen), antimikrobielle Wirkung sowie ein ebenfalls nicht persistenter sich in Richtung Superhydrophilität ändernder Kontaktwinkel festgestellt werden. Zur Herstellung des teilkristallinen Netzwerks kommt ein Brennofen bei Temperaturen von 500 bis 600 °C zum Einsatz.

Die Behandlung von Polymeren durch Plasma, Corona-Entladung und UV-Strahlung zum Zwecke der Herstellung Hydrophiler und Hydrophober Oberflächen sind aus [M. R. Wertheimer, L. Martinu, E. M. Liston,. J. Adhes. Sci. Technol., 1993, 7(10),1091.], [D. Klee, R. V. Villari, H. Höcker, B. Dekker, C. Mittermayer, J. Mat. Sci.: Mat. Med.1994, 5, 592.], [Y. I. Yoon, H. S. Moon, W. S. Lyoo, T. S. Lee, W. H. Park, J. Colloid Interface Sci. 2008, 320, 91] und [W. Xu, X. Liu, European Polymer J. 2003, 39, 199] bekannt. Es kann aber kein persistenter Effekt erzielt werden.

Der hier beschriebene Effekt kann nicht durch einmalige Einstrahlung von UV-Licht dauerhaft erzielt werden ist also nicht persistent.

Es ist Aufgabe der Erfindung, ein Verfahren zur Herstellung einer Beschichtung, die durch nachträgliche einmalige Beleuchtung in ihrer Hydrophobizität dauerhaft auf einen persistenten Wert einstellbar ist, bereitzustellen.

Weiterhin ist es Aufgabe der Erfindung ein Verfahren zur Herstellung einer Beschichtung, die durch nachträgliche einmalige Beleuchtung in ihrer Hydrophobizität auch im mikrometerskaligen Bereich dauerhaft auf einen persistenten Wert einstellbar ist, bereitzustellen.

Desweiteren ist es Aufgabe der Erfindung ein Verfahren zur Herstellung einer Beschichtung, die durch nachträgliche, einmalige Beleuchtung in ihrer Hydrophobizität dauerhaft auf einen persistenten Wert einstellbar ist, bereitzustellen, bei dem auf Temperaturen von mehr als 200 °C verzichtet wird.

Es ist darüber hinaus Aufgabe der Erfindung eine durch nachträgliche, einmalige Beleuchtung in ihrer Hydrophobizität auch im mikrometerskaligem Bereich dauerhaft auf einen persistenten Wert einstellbare, Beschichtung bereitzustellen.

Die Aufgabe wird gelöst durch das Verfahren mit den Merkmalen von Anspruch 1. Die Unteransprüche geben vorteilhafte Ausgestaltungen der Erfindung wieder.

Die Erfindung umfasst ein Herstellungsverfahren für eine Beschichtung bestehend aus den bereits bekannten Schritten zur Herstellung einer Titandioxidschicht unter Anwendung des Sol-Gel-Verfahrens:
- Herstellung einer Lösung eines Titan-Precursors in einem geeigneten Lösungsmittel
- Zugabe einer Wässrigen Lösung zur Hydrolyse des Ti-Precursors
- Herstellen einer Silberionen-haltigen Lösung in einem geeigneten Lösungsmittel
- Zugeben der Silberionenhaltigen Lösung zur Ti-haltigen Lösung
- Aufbringen auf einen geeigneten Träger
- Trocknung des Materials

### Optional sind die Verfahrens-Schritte

• Ansäuern der Ti-Precursors-Lösung
• Lagerung der Lösungen unter Kühlung
• Filtrieren

Erfindungsgemäß werden die Schritte "Herstellen einer Silberionen-haltigen Lösung in einem geeigneten Lösungsmittel" und "Trocknung des Materials" folgendermaßen variiert.

Es wird auf eine anschließende Erwärmung über 200 °C, durch Sinterung und/oder Pyrolyse verzichtet. Desweiteren werden Silberionen in einem molaren Verhältnis von Ag zu Ti von 0,2 bis 0,4 zu 1, bevorzugt von 0,25 bis 0,35 zu 1, besonders bevorzugt von 0,3 zu 1 verwendet. Die Trocknung des Materials erfolgt bei Raumtemperatur unter Ausschluss von Licht.

Bei Durchführung dieser Weiterentwicklungen der Verfahrensschritte kommt man erstmalig zu röntgenamorphen, in ihrer Hydrophobizität auf einen bestimmten Wert persistent auch im Bereich von unter 1 µm genau einstellbaren Beschichtungen (abhängig vom Strukturierungsverfahren, z.B. unter Einsatz von Elektronenstrahllithographie können submikroskopische Strukturdefinitionen erzielt werden) welche ebenfalls Gegenstand der Erfindung sind, und mit Hilfe der folgenden Figuren genauer erläutert werden. Es zeigen:
- Fig. 1: Röntgendiffraktogramme der belichteten (ir) und der unbelichteten Schicht (nir)
- Fig. 2: Elektronenmikroskopische-Aufnahmen der unbelichteten Schicht (a) (FE-SEM) und der belichteten (b) Schicht (SEM), die aus Gründen der besseren Darstellung invertiert, d.h. als Negativ aufbereitet wurden.
- Fig. 3: Benetzungsversuche: (a) belichtete (ir); (b) unbelichtete (nir) Schicht.
- Fig. 4: die Benetzung auf einer Teflonfolie im Vergleich.

Das molare Verhältnis von Ag zu Ti von 0,2 bis 0,4 zu 1, bevorzugt von 0,25 bis 0,35 zu 1, besonders bevorzugt von 0,3 zu 1 sorgt dabei für das Entstehen einer metastabilen TiO₂-AgₓO-Lösung, diese bildet wiederum ohne die übliche Erwärmung und/oder Sinterung und/oder Pyrolyse die Beschichtung.

Durch Belichtung der erfindungsgemäßen Schicht mit UV-Licht der Wellenlänge 250 nm bis 400 nm wird ein Kontaktwinkel von 30° ± 5° erreicht, dieser ändert sich auch nicht nach Abschalten der UV-Quelle und Alterung des Materials, die unbelichtete Schicht zeigt dagegen einen Kontaktwinkel von 99° ± 2°.

Es kann erstmalig der Effekt beobachtet werden, dass sich der durch Bestrahlung eingestellte Kontaktwinkel auch nach Entfernen der Strahlungsquelle nicht mehr ändert also persistent ist.

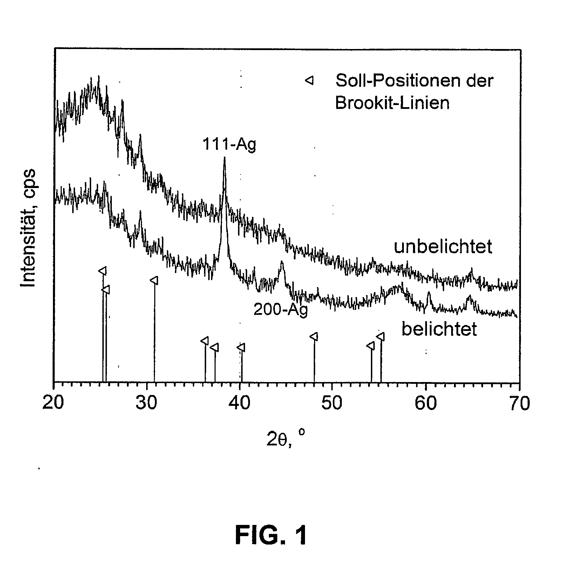

In Fig. 1 sind die Röntgendiffraktogramme der belichteten (ir) und der unbelichteten Schicht (nir) zu sehen. Die Kristallreflexe der Titandioxid-Modifikation Brookit (PDF 29-1360) sind ebenfalls eingezeichnet.

Die unbelichtete wie auch die belichtete Schicht zeigen keine Kristallreflexe des Titandioxids oder der Silberoxide, die Probe ist röntgenamorph nach der Belichtung wird lediglich das Vorhandensein von Silber, bei Abwesenheit eines Titandioxid- oder Silberoxid-Kristallnetzes angezeigt.

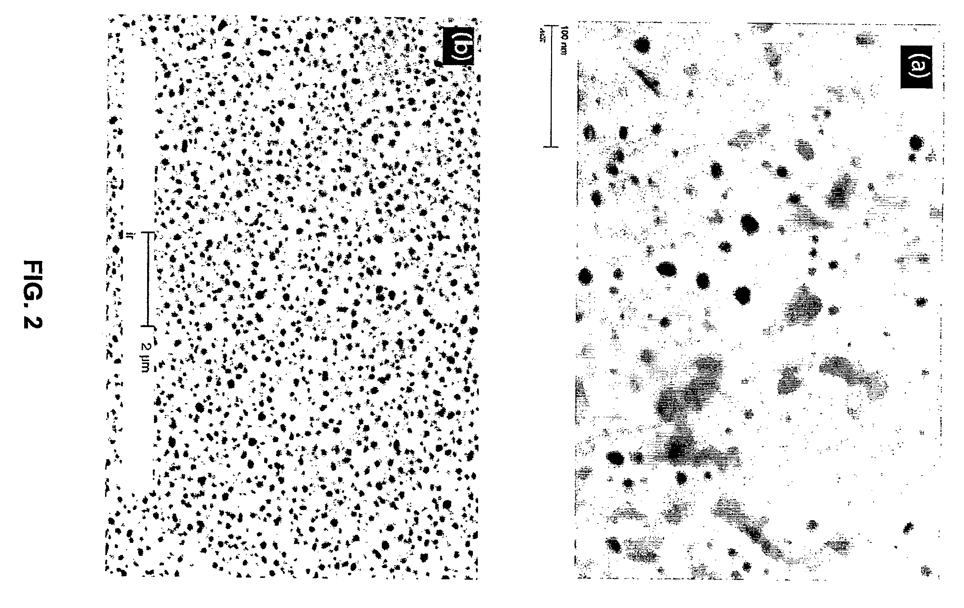

In Fig. 2 sind die elektronenmikroskopischen Aufnahmen (SEM) der unbelichteten Schicht (c) und der belichteten (d) zu sehen. Die unbelichtete Schicht zeigt nur Strukturen die deutlich kleiner als 100 nm sind, während die Belichtung zu Strukturen in der Größenordnung von ca. 100 nm führt.

Das Fehlen von Kristallstrukturen und Strukturen von einer Größe oberhalb 100 nm und die somit vorhandene Homogenität der unbelichteten, hydrophoben Schicht ermöglicht die Einstellung der Hydrophobizität durch Belichtung des Materials mit einer Genauigkeit der Grenze von 1 µm, wenn entsprechend scharf und gut platzierbare Bestrahlungsmasken aufgebracht werden. Limitierender Faktor ist hier nicht mehr die Beschichtung, sondern lediglich die sich aus der Wellenlänge des Bestrahlungslichtes ergebene Ungenauigkeit, sowie die aus der Platzierung und Beschaffenheit der Bestrahlungsmaske.

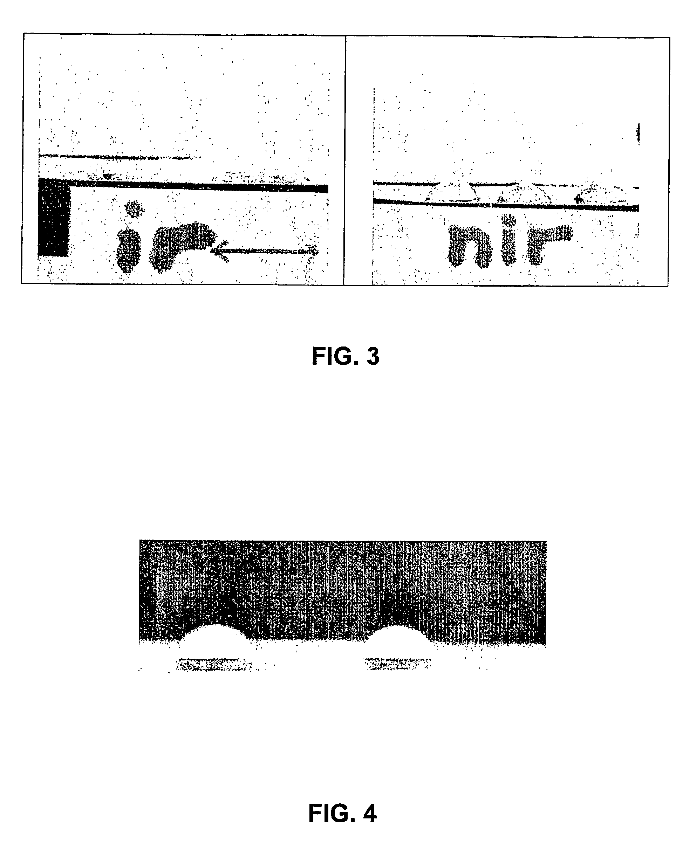

In Fig. 3 ist die Benetzbarkeit auf einer belichteten Schicht (ir) und einer unbelichteten Schicht (nir) anhand eines Wassertropfens illustriert. Fig. 4 zeigt einen Wassertropfen auf einer Teflonschicht zum Vergleich.

### Definitionen:

### Titan-Precursor:

Als Ti-Precursor kommen Alkoxide des Titans bevorzugt C₁₋₁₂-Alkoxide wie beispielsweise Tetraisoproplyorthotitanat oder Tetrabutylorthotitanat in Frage.

### Lösungsmittel:

Beispiel für geeignete Lösungsmittel sind. aliphatische Alkohole, Ether, Aldehyde, Ketone, Wasser, Ester, Alkylhalogenide aromatische Kohlenwasserstoffe, Sulfoxide, Sulfone, Amide oder deren Gemische.

### Aufbringen auf den Träger:

Das Aufbringen auf das Trägermaterial kann durch gängige nasschemische Beschichtungsverfahren erfolgen. Bespiele sind Tauchverfahren, Elektrotauchverfahren, Schleuderbeschichten, Sprühen, Spritzen, Spinnen, Ziehen, Schleudern, Gießen, Rollen, Streichen, Foliengießen, Flutbeschichten, Slot-Coating, Spin-Coating, Meniskus-Coating, Curtain-Coating, Walzenauftrag oder andere Druckverfahren.

### Bestrahlung zur Einstellung der Hydrophobizität:

Die Bestrahlung mit Elektromagnetischem Licht kann bei einer Wellenlänge von 250 nm bis 800 nm, bevorzugt ist eine Bestrahlung bei 250 nm bis 400 nm. Typische Bestrahlungsdauern liegen bei etwa 20 Minuten.

### Die Dicke der Beschichtung

Die Dicke der Beschichtung sollte mindestens 50nm betragen, besonders bevorzugt sind 300 nm bis 500 nm

### Material und Methoden

Zur Herstellung der Beschichtung wird zunächst eine TiO₂-Ag-Lösung hergestellt, die anschließend durch Tauchen, Sprühen oder Aufschleudern auf beliebige Substrate aufgebracht werden kann.

Beispielhaft wird im Folgenden die Herstellung einer TiO₂-Ag-Lösung mit einem molaren Verhältnis von Ti-Ag von 1:0,3 aufgezeigt.

Die Einwaage und Molverhältnisse sind in der nachstehenden Tabelle aufgeführt:

| Substanz | MW | mol | G | ml | Mol-verhältnis |
|---|---|---|---|---|---|
| Ti-isopropoxid | 987,9 | 0,004 | 1,136 | 1,18 | 1,00 |
| AgNO₃ | 169,9 | 0,001 | 0,216 | - | 0,30 |
| EtOH (> 99,9%) unvergällt | 46,07 | 0,306 | 18,400 | 23,29 | 76,77 |
| H₂O | 18 | 0,016 | 0,280 | 0,29 | 4,00 |
| HNO₃(65%) | 63,01 | 2,9 · 10⁻⁴ | 0,028 | 0,02 | 0,072 |

Die Herstellung erfolgt bei Raumtemperatur.

Es werden 1,18 ml Tetraisopropylorthotitanat zu 6ml Ethanol gegeben und die Mischung 10 Minuten gerührt (→ Lsg. 1). In einem weiteren Gefäß werden 0,29 ml Wasser und 6 ml Ethanol gemischt und ebenfalls 10 Minuten lang gerührt (→ Lsg. 2). Anschließend wird die wässrige Ethanollösung (Lsg 2) zur ethanolischen Tetraisopropylorthotitanat-Lösung (Lsg 1) gegeben und die Mischung 30 weitere Minuten gerührt (→ Lsg. 3). In einem weiteren Gefäß werden 0,02 ml Salpetersäure (65 %) zu 6 ml Ethanol gegeben und 10 Minuten gerührt (→ Lsg. 4). Die ethanolische Salpetersäurelösung (Lsg. 4) wird tropfenweise unter Rühren der Ti-Lösung (Lsg. 3) zugegeben anschließend wird noch 30 Minuten gerührt (→ Lsg 5). 0,216g Silbernitrat werden unter Rühren und Einsatz von Ultraschall in 5,29 ml Wasser gelöst (→ Lösung 6). Die Silbernitratlösung (Lsg. 6) wird der Ti-Lösung (Lsg. 5) zugegeben und weitere 60 Minuten gerührt.

Vor dem Aufbringen der fertigen Lösung auf die Substrate wird über ein 0,2 µm Filter filtriert. Die Lösung kann bei -40 °C gelagert werden. Vor Öffnung des gelagerten Gefäßes sollte die Lösung zunächst auf Raumtemperatur gebracht werden, um das Einkondensieren von Luftfeuchtigkeit zu verhindern.

### Bestrahlung mit UV-Licht:

Die Bestrahlung der erfindungsgemäßen Schichten erfolgt für 20 Minuten in einer abgeschlossenen Kammer mit einer 150 W UV-Lampe (Supratec UV-High Pressure lamp, Radium; 250 nm < λ < 400 nm).

### Messung des Kontaktwinkels:

Die Messung erfolgt mit einem videobasiertem Messgerät Crüss-G10, Hamburg.

### Röntgendiffraktometrie

Die Pulverdiffraktogramme (XRD) wurden unter streifendem Einfall (GI) mit Hilfe eines Seifert 3000 PTS 4-circle-Diffractometer, mit Cu Kα Linie (λ = 1,5418 Å) aufgenommen. Der GI-Winkel lag bei 5°.

### Elektronenmikroskopie:

Die SEM-Aufnahmen wurden mit einem (Philips XL 30) ausgestattet mit Energie- dispersiver Röntgenspektroskopie (EDS, EDAX CDU Leap Detektor) die FE-SEM mit einem Schottky emitter (FE-SEM , Zeiss Ultraplus, Germany) gemacht.

## Patentansprüche

1. Substrat mit einer Silberionen enthaltenden Titanoxidbeschichtung mit einem Silbergehalt von größer oder gleich 0,2 mol Ag / 1 mol Ti bis kleiner oder gleich 0,4 mol Ag / 1 mol Ti, wobei die Beschichtung röntgenamorph und die Hydrophobizität der Beschichtung durch Beleuchtung persistent herabsetzbar ist.

2. Substrat nach Anspruch 1, **dadurch gekennzeichnet, dass** die Hydrophobizität der Beschichtung durch Beleuchtung bei einer Wellenlänge von 250 nm bis 400 nm persistent herabsetzbar ist.

3. Substrat nach einem der Ansprüche 1 und 2, **dadurch gekennzeichnet, dass** die Beschichtung bei einer Temperatur gleich oder weniger 200 °C getrocknet wurde.

4. Substrat nach einem der Ansprüche 1 bis 3, **dadurch gekennzeichnet, dass** die Beschichtung eine Dicke von 300 bis 500 nm hat.

5. Verfahren zum Herstellen einer röntgenamorphen Beschichtung mit einer durch Beleuchtung persistent herabsetzbaren Hydrophobizität auf einem Substrat, mit den Schritten:
- Herstellen einer Titan-Precursor-Lösung,
- Hydrolysieren des Ti-Precursors,
- Zugeben einer Silberionenhaltigen Lösung zur Titan-haltigen Lösung in einem molaren Verhältnis von 0,2 mol Ag / 1 mol Ti bis 0,4 mol Ag / 1 mol Ti,
- Beschichten eines Trägermaterials mit der TiO₂-Ag-Lösung
- Trocknen der auf den Träger aufgebrachten Beschichtung bei einer Temperatur gleich oder weniger 200 °C, und
- Trocknen der auf den Träger aufgebrachten Beschichtung unter Ausschluss von Licht.

6. Verfahren nach Anspruch 5, **dadurch gekennzeichnet, dass** die Titan-Precursor-Lösung einen pH-Wert von < 7 aufweist.

7. Verfahren nach einem der Ansprüche 5 und 6, **dadurch gekennzeichnet, dass** die TiO₂-Ag-Lösung durch ein Filter mit einer Maschenweite von 0,2 µm filtriert wird.

8. Verfahren nach einem der Ansprüche 5 bis 7, **dadurch gekennzeichnet, dass** die TiO₂-Ag-Lösung kühl gelagert wird.

9. Verfahren nach einem der Ansprüche 5 bis 8, **dadurch gekennzeichnet, dass** das Licht Licht einer Wellenlänge von 250 nm bis 400 nm ist.

10. Verfahren nach einem der Ansprüche 5 bis 9, **dadurch gekennzeichnet, dass** das Beleuchten bis 20 min andauert.

11. Verfahren nach einem der Ansprüche 5 bis 10, **dadurch gekennzeichnet, dass** die Beschichtung eine Dicke von 300 bis 500 nm hat.

## Claims

1. A substrate having a silver-ion-containing titanium oxide coating with a silver content of at least 0.2 mol Ag / 1 mol Ti up to at most 0.4 mol Ag / 1 mol Ti, the coating being x-ray amorphous and it being possible for the hydrophobicity of the coating to be reduced persistently by illumination.

2. The substrate according to Claim 1, **characterized in that** the hydrophobicity of the coating can reduced persistently by illumination at a wavelength of 250 nm to 400 nm.

3. The substrate according to one of Claims 1 and 2, **characterized in that** the coating was dried at a temperature of at most 200 °C.

4. The substrate according to one of Claims 1 to 3, **characterized in that** the coating has a thickness of 300 to 500 nm.

5. A method for producing an x-ray amorphous with a hydrophobicity that can be reduced persistently by illumination on a substrate, having the following steps:
- producing a titanium precursor solution,
- hydrolysing the Ti precursor,
- adding a silver-ion-containing solution to the titanium containing solution in a molar ratio of 0.2 mol Ag / 1 mol Ti to 0.4 mol Ag / 1 mol Ti,
- coating a carrier material using the TiO₂-Ag solution
- drying the coating applied to the carrier at a temperature of at most 200 °C, and
- drying the coating applied to the carrier without light.

6. The method according to Claim 5, **characterized in that** the titanium precursor solution has a pH of < 7.

7. The method according to one of Claims 5 and 6, **characterized in that** the TiO₂-Ag solution is filtered through a filter having a mesh width of 0,2 µm.

8. The method according to one of Claims 5 to 7, **characterized in that** the TiO₂-Ag solution is stored in a cool place.

9. The method according to one of Claims 5 to 8, **characterized in that** the light is light at a wavelength of 250 nm to 400 nm.

10. The method according to one of Claims 5 to 9, **characterized in that** the illumination lasts up to 20 min.

11. The method according to one of Claims 5 to 10, **characterized in that** the coating has a thickness of 300 to 500 nm.

## Revendications

1. Substrat avec un revêtement en oxyde de titane contenant des ions d'argents avec une teneur en argent étant égale ou supérieure à 0,2 mol Ag / 1 mol Ti jusqu'à inférieure ou égale à 0,4 mol Ag / 1 mol Ti, le revêtement étant amorphe au rayon X et l'hydrophobie du revêtement pouvant être diminuée de manière persistante par illumination.

2. Substrat selon la revendication 1, **caractérisé par le fait que** l'hydrophobie du revêtement peut être diminuée de manière persistante par une illumination avec une longueur d'onde entre 250 nm et 400 nm.

3. Substrat selon une des revendications 1 et 2, **caractérisé par le fait que** le revêtement à été séché à une température inférieure ou égale à 200 °C.

4. Substrat selon une des revendications 1 à 3, **caractérisé par le fait que** le revêtement dispose d'une épaisseur entre 300 et 500 nm.

5. Procédé pour la fabrication d'un revêtement amorphe aux rayons X avec une hydrophobie pouvant être diminuée de manière persistante sur un substrat, par les étapes :
- Fabrication d'une solution Titan précurseur
- Hydrolyser les précurseurs Ti
- Ajout d'une solution contenant des ions d'argent à la solution contenant du Titan avec rapport molaire de 0,2 mol Ag / 1 mol Ti jusqu'à 0,4 mol Ag / 1 mol Ti,
- Recouvrir un matériau support avec la solution TiO₂-Ag
- Séchage du revêtement appliqué sur le support à une température égale ou inférieure à 200 °C, et
- Séchage du revêtement appliqué sur le support à l'abri de la lumière.

6. Procédé selon revendication 5, **caractérisé par le fait que** la solution Titan précurseur affiche un pH < 7.

7. Procédé selon une des revendications 5 et 6, **caractérisé par le fait que** la solution TiO₂-Ag est filtrée par un filtre avec une finesse de 0,2 µm.

8. Procédé selon une des revendications 5 à 7, **caractérisé par le fait que** la solution TiO₂-Ag est conservée au frais.

9. Procédé selon une des revendications 5 à 8, **caractérisé par le fait que** la lumière est une lumière avec une longueur d'onde entre 250 et 400 nm.

10. Procédé selon une des revendications 5 à 9, **caractérisé par le fait que** l'illumination dure jusqu'à 20 min.

11. Procédé selon une des revendications 5 à 10, **caractérisé par le fait que** le revêtement dispose d'une épaisseur entre 300 et 500 nm.
